# EUROPÄISCHE PATENTANMELDUNG

(11) **EP 2 201 942 A1**
(43) Veröffentlichungstag der Anmeldung: **30.06.2010**
(21) Anmeldenummer: 08075967.3
(22) Anmeldetag: 23.12.2008
(51) Int. Cl.: A61K 31/00, A61K 36/185, A61K 9/00, A61P 17/00

(54) **Zusammensetzung mit Hanföl für die Behandlung topischer Erkrankungen**

(71) Anmelder: Intendis GmbH, 10589 Berlin (DE)
(72) Erfinder: Schmidt, Timm, 10117 Berlin (DE)
(74) Vertreter: Seuss, Thomas

(57) **Zusammenfassung**

Die Erfindung betrifft eine neue Zusammensetzung, enthaltend Hanföl zur Behandlung topischer Erkrankungen, insbesondere des Hand-Fuß-Syndroms.

## Beschreibung

### Stand der Technik

Bei der Anwendung von Zytostatika (Chemotherapeutika wie beispielsweise 5-FluorUracil (5-FU), Gemcitabin, Methotrexat, und andere) in der Krebstherapie kommt es bei Patienten häufig zur Ausbildung des sogenannten Hand-Fuß-Syndroms. Es handelt sich dabei um eine palmar-plantare Erythrodysästhesie. Diese Hautläsion kann den Patienten leicht bis sehr schwer in seinem täglichen Leben beeinträchtigen. Die Pathogenese der Erkrankung ist weitgehend unklar. (Salzberg et al, Schweiz Med. Wochenschr. 2000; 130: 1413-6). Die Behandlung des Hand-Fuß-Syndroms erfolgt beispielsweise durch Gabe von Schmerzmitteln, Cortikosteroiden, D-Panthenol oder Vitamin B 6. Weiterhin werden Dosisanpassungen des jeweiligen Zytostatikums vorgeschlagen (Salzberg et al, siehe oben).

Zur Behandlung des Hand-Fuß-Syndroms als Nebenwirkung bei der Behandlung mit Capecitabine wurde weiterhin eine Hanfölsalbe vorgeschlagen. Diese Salbe wurden auf Basis von Hanföl unter Zusatz von verschiedenen Pflanzenfetten w. z. B. Mangobutter und Sheabutter sowie Bienenwachs mit weiteren Zusätzen von D-Panthenol, Harnstoff, Bienenhonig, Lavendelöl und Aloe-Vera-Gel hergestellt (F.Kober, www.aekwien.at/media/BAEZ_6Bez_modernetherapiekonzepte.pdf, undatiert, heruntergeladen: 22.12.2008). Die Salbe weist allerdings nur eine geringe Lagerfähigkeit (< 3 Monate) auf. Die Wirksamkeit der Zubereitung nimmt mit der Zeit ab. Weiterhin weisen derartige Salben einen unangenehmen Geruch auf, der den Patienten die regelmäßige Anwendung verleidet.

Es besteht daher Bedarf an neuen Zubereitungen zur Behandlung des Hand-Fuß-Syndroms, die die Nachteile des Standes der Technik überwinden.

### Allgemeine Beschreibung der Erfindung

Es wurde nun gefunden, dass eine Zubereitung enthaltend Hanföl und mindestens ein Antioxidans die Nachteile des Standes der Technik überwindet und eine Zusammensetzung zur Verfügung stellt, die (unter anderem) für die Behandlung des Hand-Fuß-Syndroms hervorragend geeignet ist.

Bei dem verwendeten Hanföl handelt es sich um aus Hanfsamen gewonnenes Hanföl. Dieses enthält kein Tetrahydrocannabinol (THC).
Es enthält allerdings eine Reihe ungesättigter Fettsäuren, wie beispielsweise Linolsäure, Alphalinolensäure und Gammalinolensäure. Für die erfindungsgemäße Zusammensetzung ist entscheidend, dass das Verhältnis von Linolsäure + Gammalinolensäure zu Alphalinolensäure ungefähr 3:1 ist.
Der Anteil des Hanföls in der erfindungsgemäßen Zusammensetzung beträgt etwa 10 - 40 Gewichtsprozent, bevorzugt 25 - 35 Gewichtsprozent, ganz besonders bevorzugt 30 Gewichtsprozent.

Von besonderer Bedeutung für die erfindungsgemäße Zusammensetzung ist die Anwesenheit mindestens eines Antioxidans. Dieses kann beispielsweise ausgewählt sein aus Ascorbinsäure, Vitamin E (insbesondere Tocotrienole), Carotinoide (insbesondere Lycopin), Glutathion, Butylhydroxytoluol und Butylhydroxyanisol oder eine Mischung der genannten Substanzen.
Das bevorzugte Antioxidans ist Butylhydroxytoluol. Das Antioxidans wird im Allgemeinen in einem Anteil von 0,01 - 0,5 Gewichtsprozent, bevorzugt 0,025 - 0,075 Gewichtsprozent, insbesondere bevorzugt ca. 0,05 Gewichtsprozent in der Zusammensetzung enthalten sein.

Optional kann noch ein weiteres Konservierungsmittel enthalten sein. Das Konservierungsmittel kann dabei aus der folgenden Gruppe ausgewählt sein: Phenoxyethanol, Methylparaben, Ethylparaben, Propylparaben, Benzylalkohol oder eine Mischung der genannten Mittel.
Bevorzugt wird das Konservierungsmittel Phenoxyethanol in einem Anteil von 0,2 - 2 Gewichtsprozent, besonders bevorzugt 0,5 - 1,5 Gewichtsprozent, ganz besonders bevorzugt ca. 1 Gewichtsprozent zugegeben.

Die erfindungsgemäße Zusammensetzung wird bevorzugt als Creme hergestellt.

Eine besonders bevorzugte Ausführungsform der erfindungsgemäßen Zusammensetzung ist eine Creme mit folgender Zusammensetzung:

| | |
|---|---|
| Cetearyl Alcohol / Cetearyl Glucoside | ca. 7 g/100 g |
| Hanföl | ca. 30 g/100 g |
| Phenoxyethanol | ca. 1g/100g |
| Butylhydroxytoluol | ca. 0,05 g/100g |

welche mit Milchsäure auf einen pH Wert von ca. 5,5 eingestellt und mit Wasser auf 100g aufgefüllt wird.

Die erfindungsgemäße Zusammensetzung kann für die topische Behandlung menschlicher Haut verwendet werden. Der Begriff Behandlung umfasst dabei die kosmetische, wie auch die therapeutische Behandlung der menschlichen Haut. Ferner ist auch die prophylaktische Anwendung umfasst.

Von besonderer Bedeutung ist die Verwendbarkeit der erfindungsgemäßen Zusammensetzung für die Behandlung des Hand-Fuß-Syndroms. Betroffene Patienten spüren bereits nach kurzer Zeit eine Linderung ihrer Beschwerden, die oftmals durch Anwendung der erfindungsgemäßen Zusammensetzung ganz verschwinden.

Bei prophylaktischer Anwendung der erfindungsgemäßen Zusammensetzung während der Anwendung von Zytostatika wird eine signifikante Verringerung der Anzahl von Patienten beobachtet, die an Hand-Fuß-Syndrom leiden. Gegenstand der Erfindung ist daher auch ein pharmazeutisches Kit, enthaltend ein Chemotherapeutikum und eine Zusammensetzung gemäß mindestens einem der Ansprüche 1-9. Als Kit wird hierbei ein Gebinde bezeichnet, welches ein Chemotherapeutikum und eine Zusammensetzung gemäß mindestens einem der Ansprüche 1-9 enthält, wobei das Chemotherapeutikum und die Zusammensetzung gemäß mindestens einem der Ansprüche 1-9 getrennt verpackt sein kann, aber gemeinsam an den Patienten oder den Arzt bzw. die Klinik abgegeben wird.
Ein bevorzugter Gegenstand der Erfindung ist ein pharmazeutisches Kit, bei dem das Chemotherapeutikum ausgewählt ist aus Actinomycin D, Actinomycine, Alemtuzumab, Alkylantien, Allicin, Aminopterin, Anthracycline, Asparaginase, Atrasentan, Azacitidin, Azathioprin, Bevacizumab. Bleomycin, Bortezomib, Bosutinib, Busulfan, Capecitabin, Carboplatin, Carmustin, Cetuximab, Chlorambucil, Cilengitid, Cisplatin, Cladribin, Colchicin, Cyclophosphamid, Cytarabin, Dasatinib, Daunorubicin, Decitabin, Docetaxel, Doxifluridin, Doxorubicin, Epirubicin, Epothilone, Erlotinib, Estramustin, Etoposid, Fludarabin, 5-Fluorouracil, Folsäure-Analoga, Gemcitabin, Gendicine, Hydroxycarbamid, INNO-406, Ibritumomab-Tiuxetan, Ifosfamid, Imatinib, Immucillin-H, Irinotecan, Lapatinib, Lomustin, Matuzumab, Mechlorethamin, Mercaptopurin, Methotrexat, Miltefosin, Mitomycin, Mitoxantron, Nilotinib, Nimustin, Oxaliplatin, Paclitaxel, Panitumumab, Pemetrexed, Podophyllotoxin, Procarbazin, Rituximab, Sorafenib, Stimuvax, Streptozocin, Temozolomid, Teniposid, Thiotepa, Tioguanin, Topotecan, Trastuzumab, Triptolid, Vinblastin, Vincaalkaloide, Vincristin, Vindesin, Vinorelbin, Zinostatin,
in Kombination mit einer Zusammensetzung gemäß mindestens einem der Ansprüche 1-9.

Ein besonders bevorzugter Gegenstand der Erfindung ist ein pharmazeutisches Kit, bei dem Sorafenib, in Kombination mit einer Creme mit folgender Zusammensetzung enthalten ist:

| | |
|---|---|
| Cetearyl Alcohol / Cetearyl Glucoside | ca. 7 g / 100 g |
| Hanföl | ca. 30 g / 100 g |
| Phenoxyethanol | ca. 1 g /100 g |
| Butylhydroxytoluol | ca. 0,05 g /100 g |

welche mit Milchsäure auf einen pH Wert von ca. 5,5 eingestellt und mit Wasser auf 100g aufgefüllt wird.

Überraschenderweise wurde darüberhinaus gefunden, dass die erfindungsgemäße Zusammensetzung auch für die Behandlung der Psoriasis und der Neurodermitis geeignet ist. Gegenstand der Erfindung ist daher auch die Verwendung der erfindungsgemäßen Zusammensetzung zur Behandlung dieser Hautkrankheiten.

### Herstellung

Die Creme wird nach Standardmethoden der pharmazeutischen Technologie hergestellt. Diese sind dem Fachmann bekannt und benötigen keine weitere Erläuterung.

### Beispiele

### Beispiel 1

Die pharmazeutische Zusammensetzung in Form einer Creme wird, in üblicher, dem Fachmann bekannter Weise, in den nachfolgend angegebenen Zusammensetzungen (A - E) angerührt. Der pH-Wert wird jeweils mit Milchsäure auf 5,5 eingestellt.

| **Bestandteil** | Menge [g/100g] | Menge [g/100g] | Menge [g/100g] | Menge [g/100g] | Menge [g/100g] |
|---|---|---|---|---|---|
| | **A** | **B** | **c** | **D** | **E** |
| Cetearyl Alcohol / Cetearyl Glucoside | 7 | 7 | 8 | 7 | 6 |
| Hanföl | 30 | 25 | 35 | 30 | 30 |
| Phenoxyethanol | 1 | 1 | 1 | 1 | 1 |
| Butylhydroxytoluol | 0,05 | 0,05 | 0,05 | 0,01 | 0,10 |
| Wasser | ad 100 | ad 100 | ad 100 | ad 100 | ad 100 |

### Beispiel 2

Die Zusammensetzungen gem. Beispiel 1 A - E werden über längere Zeit bei erhöhter Temperatur eingelagert. Es wird festgestellt, dass die erfindungsgemäße Zusammensetzung eine Lagerfähigkeit von deutlich über 2 Jahren aufweist.

### Beispiel 3

Patienten mit Hand-Fuß-Syndrom werden mit der erfindungsgemäßen Zusammensetzung behandelt. Die Anwendung erfolgt mehrmals täglich, bevorzugt mindestens dreimal täglich auf den betroffenen Stellen. Die Patienten zeigen bereits nach einwöchiger Anwendung eine deutliche Linderung ihrer Symptomatik, zum Teil verschwinden die Symptome vollständig nach einer Woche.

### Beispiel 4

Krebspatienten, die mit Sorafenib (INN, Handelsname Nexavar^{®}) behandelt werden sollen, erhalten zeitgleich mit der ersten Gabe von Sorafenib die erfindungsgemäße Zusammensetzung. Die Anwendung der erfindungsgemäßen Zusammensetzung erfolgt mehrmals täglich, bevorzugt mindestens dreimal täglich auf den betroffenen Stellen. Die Patienten zeigen eine signifikant verringerte Tendenz zur Ausbildung des Hand-Fuß Syndromes.

### Beispiel 5

Patienten mit Neurodermitis werden mehrfach täglich mit der erfindungsgemäßen Zusammensetzung behandelt. Bereits nach einer Woche Anwendung ist eine deutliche Linderung der Beschwerden feststellbar.

## Patentansprüche

1. Zusammensetzung für die topische Behandlung menschlicher Haut in einem dermatologisch verträglichen Träger, enthaltend
(a) Hanföl
(b) mindestens ein Antioxidans.

2. Zusammensetzung gemäß Anspruch 1 in Form einer Creme.

3. Zusammensetzung gemäß Anspruch 1, worin das Hanföl in einem Anteil von 10-40 Gewichtsprozent, bevorzugt 25-35 Gewichtsprozent vorliegt.

4. Zusammensetzung gemäß Anspruch 1, worin das Hanföl in einem Anteil von ca. 30 Gewichtsprozenten vorliegt.

5. Zusammensetzung gemäß Anspruch 1, worin das Antioxidans ausgewählt ist aus Ascorbinsäure, Vitamin E (insbesondere Tocotrienole), Carotinoide (insbesondere Lycopin), Glutathion, Butylhydroxytoluol und Butylhydroxyanisol oder einer Mischung der genannten Mittel.

6. Zusammensetzung gemäß Anspruch 5, worin das Antioxidans Butylhydroxytoluol ist, welches in einem Anteil von 0,01 - 0,5 Gewichtsprozent, bevorzugt 0,025-0,075 Gewichtsprozent, besonders bevorzugt ca. 0,05 Gewichtsprozent vorliegt.

7. Zusammensetzung gemäß Anspruch 1, worin zusätzlich ein Konservierungsmittel enthalten ist, welches ausgewählt ist aus Phenoxyethanol, Methylparaben, Ethylparaben, Propylparaben, Benzylalkohol oder einer Mischung der genannten Mittel.

8. Zusammensetzung gemäß Anspruch 1, worin das Konservierungsmittel Phenoxyethanol ist, welches in einem Anteil von 0,2 - 2,0 Gewichtsprozent, bevorzugt 0, 5-1,5 Gewichtsprozent, besonders bevorzugt ca. 1,0 Gewichtsprozent vorliegt.

9. Zusammensetzung gemäß Anspruch 1 in Form einer Creme mit folgender Zusammensetzung:
| | |
|---|---|
| Cetearyl Alcohol / Cetearyl Glucoside | ca. 7 g / 100 g |
| Hanföl | ca. 30 g / 100 g |
| Phenoxyethanol | ca.1g/100g |
| Butylhydroxytoluol | ca. 0,05 g / 100g |
welche mit Milchsäure auf einen pH Wert von ca. 5,5 eingestellt und mit Wasser auf 100g aufgefüllt wird.

10. Verwendung einer Zusammensetzung gemäß mindestens einem der Ansprüche 1-9 zur topischen Behandlung menschlicher Haut.

11. Verwendung gemäß Anspruch 10 zur Behandlung des Hand-Fuß-Syndromes, der Psoriasis oder der Neurodermitis.

12. Pharmazeutisches Kit, enthaltend ein Chemotherapeutikum und eine Zusammensetzung gemäß mindestens einem der Ansprüche 1-9.

13. Pharmazeutisches Kit, enthaltend Sorafenib und eine Zusammensetzung gemäß Anspruch 9.
